Europäisches Patentamt

European Patent Office

Office européen des brevets

(11) Veröffentlichungsnummer: **0 001 608**
**B1**

(12) **EUROPÄISCHE PATENTSCHRIFT**

(45) Veröffentlichungstag der Patentschrift:
**22.12.82**

(21) Anmeldenummer: **78101107.7**

(22) Anmeldetag: **09.10.78**

(51) Int. Cl.³: **G 01 S 7/62,** A 61 B 10/00,
H 04 N 5/20

(54) **Ultraschall-Bildgerät.**

(30) Priorität: **21.10.77 DE 2747405**

(43) Veröffentlichungstag der Anmeldung:
**02.05.79 Patentblatt 79/9**

(45) Bekanntmachung des Hinweises auf die Patenterteilung:
**22.12.82 Patentblatt 82/51**

(84) Benannte Vertragsstaaten:
**CH DE**

(56) Entgegenhaltungen:
**US-A-2 884 484**
**US-A-3 588 338**
**US-A-3 975 704**

JOURNAL OF PHYSICS E(SCIENTIFIC INSTRU-MENTS) vol. 9, no. 3, (März 1976), London GB HILL: «Ultrasonic Imaging», Seiten 153–162.

ULTRASONICS, vol. 10, no. 5, (September 1972) London GB KOSSOFF: «Improved techniques in ultrasonic bross sectional echography», Seiten 221–227.

(73) Patentinhaber: **SIEMENS AKTIENGESELLSCHAFT**
**Berlin und München, Postfach 22 02 61,**
**D-8000 München 22 (DE)**

(72) Erfinder: **Walz, Alfred, Tannenweg 14, D-8501 Burgthann (DE)**
Erfinder: **Birk, Adalbert, Paul-Gossen-Strasse 34, D-8520 Erlangen (DE)**

## Ultraschall-Bildgerät

Die Erfindung bezieht sich auf ein Ultraschall-Bildgerät für Ultraschall-Echoaufzeichnung nach dem Impuls-Echo-Prinzip, mit Ultraschall-Sender/Empfänger, der Echosignale mit bestimmtem Dynamikbereich liefert, sowie einer Bildaufzeichnungsvorrichtung für die Echosignale, die eine nichtlineare Leuchtdichte/Ansteuer-Kennlinie aufweist.

Das Ultraschall-Bildverfahren bringt neben den Oberflächenkonturen der Organe und Gefässe auch die feinen Strukturen innerhalb der Organe oder Gefässe zur Darstellung. Die Qualität eines Ultraschallbildes ist nun nicht nur durch die physikalische Punktauflösung im Ultraschall-Strahl bestimmt; sie wird vielmehr auch durch die Art der Amplitudenwiedergabe auf dem Bildschirm der Oszillographenröhre oder dem Foto des abfotografierten Sichtbildes beeinflusst. Echosignale, die sich in ihrer Amplitude unterscheiden, werden auf dem Bildschirm als Bildpunkte unterschiedlicher Leuchtdichte dargestellt. Leuchtdichteunterschiede werden entsprechend vom Auge des Beobachters als Unterschiede im Grauwert wahrgenommen. Die Amplituden der aus dem Körperinnern empfangenen Echos umfassen einen Dynamikbereich von mehr als 100 dB («maximaler Echokontrast»). Dabei unterscheiden sich die durch diffuse Streuung entstehenden Strukturechos durch ihre kleinere Amplitude von den im wesentlichen durch spiegelnde Reflexion erzeugten Konturechos, die durchweg grössere Amplitude aufweisen. Dem Echodynamikbereich von ca. 100 dB steht nun ein Dynamikbereich der Leuchtdichte von nur maximal 30 dB gegenüber, den das adaptierte Auge des Beobachters in Grauwerte zwischen schwarz und weiss auflösen kann («maximaler Bildkontrast»). Nach dem Weber-Fechner-Gesetz bewertet das Auge die innerhalb dieses Bereiches wahrgenommene Leuchtdichte logarithmisch. Der kleinste vom Auge noch unterscheidbare Leuchtdichtenunterschied ist der Schwellenkontrast $d_0$; er beträgt $d_0 = 0{,}83$ dB. Demnach löst das adaptierte Auge des Beobachters den 30 dB-Bereich in ca. 35 Graustufen auf. Diese sinnesphysiologischen Gegebenheiten sind für die Sonographie in zweierlei Hinsicht von Bedeutung. Zum einen kann das Auge aus dem grossen Echodynamikbereich nur einen Ausschnitt erfassen. Dieser Ausschnitt kann mit Hilfe eines Verstärkungsreglers ausgewählt und die Grösse des Ausschnittes dann durch Dynamikkompression beeinflusst werden. Zum zweiten ist die Strukturauflösung im Ultraschallbild nicht allein durch die als Halbwertsbreite des Echosignals eines Punktobjektes definierte physikalische Lateralauflösung bestimmt; sie ist vielmehr auch durch den Schwellenkontrast der Grauwertauflösung beeinflusst. So sind beispielsweise zwei Punktobjekte mit dem lateralen Abstand A im Ultraschallbild voneinander unterscheidbar, solange $d_1 - d_2 \geq n \cdot d_0$ mit $d_0 = 0{,}83$ dB ist. Dabei ist $d_1$ das Echosignal, das entsteht, wenn einer der beiden Objekte auf der Strahlachse liegt, und $d_2$ ist das entsprechende Signal bei einem Abstand A/2 der beiden Objekte von der Strahlachse. n ist der Kompressionsfaktor für den Fall einer Dynamikkompression der Form: Leuchtdichte ~ (Echoamplitude)$^{1/n}$. Anstelle der (−6 dB)-Breite des Echosignals eines Punktobjekts ist die laterale Auflösung demnach durch die (−6− n · 0,83)dB-Breite gegeben. Diese Definition der Punktauflösung ist nur gültig und invariant beispielsweise gegenüber der Wahl der Echoverstärkung, solange der Zusammenhang zwischen Echoamplitude und Leuchtdichte linear ist oder der oben gegebenen Potenzfunktion folgt. Die Definition verliert ihre Gültigkeit, wenn das Übertragungssystem einer davon abweichenden, z. B. logarithmischen, Funktion folgt.

Aufgabe vorliegender Erfindung ist es, ein Ultraschall-Bildgerät mit einer solchen Signalübertragungskette aufzubauen, mit der sich in einfacher Weise der Echodynamikbereich an den vom Auge erfassbaren Leuchtdichtebereich anpassen lässt.

Die Aufgabe wird unter Berücksichtigung der oben angeführten Überlegungen dadurch gelöst, daß zwischen Ultraschallempfänger und Bildaufzeichnungsvorrichtung ein Verzerrglied ähnlich oder gleich einem γ-Korrekturglied, wie es an sich aus der Fernsehtechnik vorbekannt ist, eingeschaltet ist, das die nichtlineare Leuchtdichte/Ansteuer-Kennlinie der Aufzeichnungsvorrichtung in Abhängigkeit von einem vorgebbaren optimalen Dynamikbereich der Echosignale nach vorgebbarem Muster verzerrt, insbesondere linearisiert, und dass dem Verzerrglied ein Schwellenregler vorgeschaltet ist, der in Abhängigkeit von einer vorgebbaren Schwellenspannung, die oberhalb der Amplitude von Störechos liegt, solche Signalamplituden, die die Schwellspannung überschreiten, intensitätsgetreu, jedoch unter Unterdrükkung der Störechos zum Verzerrglied führt, wozu der Schwellenregler einen Komparator mit zugeordnetem Schalttransistor umfasst, wobei der Transistor bei Signalen unterhalb der am Komparator eingestellten Schwelle einen Kurzschluss für das Eingangssignal bildet, der jedoch aufgehoben wird, sobald das einkommende Signal amplitudenmässig die Schwelle überschreitet.

Auf diese Weise lässt sich beispielsweise bei Verwendung einer Bildröhre die Dynamikdilatation der Röhre kompensieren in dem Sinne, dass einem durch den Empfangsverstärker bereits auf 30 dB eingeschränkten Echodynamikbereich ein maximaler Leuchtdichtebereich von ebenfalls 30 dB entspricht. γ-Korrekturglieder in Verbindung mit Fernsehtechnik sind dabei z. B. durch die US-PS-28 84 484 und US-PS-35 88 338 bekannt. Zusätzlich zum Korrekturglied ist aber auch noch ein Schwellenregler mit den vorstehend beschriebenen Merkmalen vorgesehen. Ein solcher Schwellenregler erlaubt die Elimination von kleinen Störechos, wobei jedoch Nutzechos, die über der Schwelle des Schwellenreglers liegen, in ihrer Amplitude und damit auch in der Echograuwertdarstellung im Sichtbild völlig unberührt bleiben. Die Kombination beider Massnahmen ermöglicht also den Aufbau einer Signalübertragungskette, mit der sich in optimaler Weise der Echodynamikbereich an den vom Auge erfassbaren Leuchtdichtebereich anpassen lässt.

Weitere Vorteile und Einzelheiten der Erfindung ergeben sich aus der nachfolgenden Beschreibung eines Ausführungsbeispiels anhand der Zeichnung in Verbindung mit den Unteransprüchen.

Es zeigen:

Figur 1 die Signalübertragungskette eines Ultraschall-Bildgerätes im Prinzipschaltbild mit Linearsierglied als Verzerrglied,

Figur 2 eine Ausführungsform für einen Schwellenregler,

Figur 3 eine Ausführungsform für ein Linearsierglied,

Figur 4 einen Kontrastregler für den Bildkontrast an der Bildröhre.

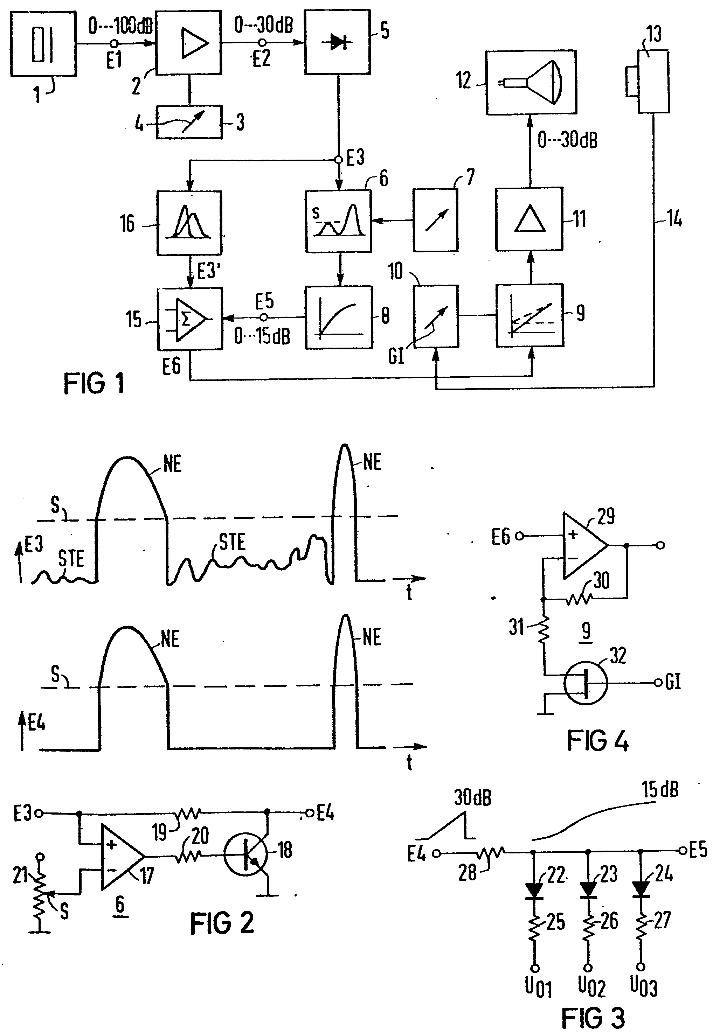

In der Fig. 1 ist ein Ultraschallwandler zum Aussenden von Ultraschallimpulsen und zum Empfang der Echoimpulse mit 1 bezeichnet. Der Wandler 1 kann dabei ein Rotationswandler sein, der in Verbindung mit einem Parabolreflektor Zeilenabtastung eines Untersuchungsobjektes ermöglicht. Es kann sich aber auch um ein Ultraschall-Array handeln, wo also eine Vielzahl nebeneinander angeordneter Ultraschallschwinger zur zeilenweisen Objektabtastung dient. Ebensogut kann es auch ein Wandler für Compound-Scan- oder aber A-Scan-Betrieb sein. Die vom Wandler 1 empfangenen Echosignale E1, die einen Echodynamikbereich von 0 bis etwa 100 dB aufweisen, werden mittels Echosignalverstärker 2 verstärkt und im Dynamikbereich gleichzeitig auf 0 bis 30 dB eingeschränkt. Zur Einstellung eines erwünschten Verstärkungsgrades am Echosignalverstärker 2 dient der Verstärkungsregler 3 mit Einstellglied 4. Die am Ausgang des Echosignalverstärkers 2 anfallenden dynamikbegrenzten Echosignale E2 werden mittels Gleichrichter 5 (Diodengleichrichter) demoduliert und das demodulierte Signal E3 auf einen Schwellenregler 6 mit Schwelleneinstellglied 7 gegeben. Der Schwellenregler 6 unterdrückt kleine Störechos, die unterhalb der Schwelle S liegen. Nutzechos, die die Schwelle überschreiten, werden hingegen in der Amplitude unverzerrt hindurchgelassen. Am Ausgang des Schwellenreglers fällt demnach ein Echosignal E4 an, aus dem kleine Störechos völlig eliminiert sind. Das so bearbeitete Echosignal E4 wird schliesslich auf ein γ-Korrekturglied 8 gegeben, das eine zur γ-Kurve der Bildröhre inverse Kennlinie aufweist. Durch die Kompensation wird der Dynamikbereich des Echosignals E4 auf maximal 15 dB eingegrenzt und es ergibt sich somit aufgrund des quadratischen Kennlinienverlaufs der Bildröhre ein maximaler Leuchtdichtebereich von 30 dB. Würde das auf 30 dB begrenzte Echosignal direkt zur Strahlstromsteuerung der Bildröhre benutzt, so würde wegen der quadratischen Kennlinie (γ-Kurve) der Röhre der korrespondierende Leuchtdichte-Dynamikbereich, d.h. der maximale Bildkontrast, 60 dB betragen. Dieser Kontrast kann jedoch das menschliche Auge, wie eingangs bereits erläutert, nicht auflösen. Durch Zwischenschaltung des γ-Korrekturgliedes 8 wird hingegen die Dynamikdilatation der Röhre völlig kompensiert, so dass also dem ursprünglichen 30 dB-Bereich des Echosignals E2 am Ausgang des Empfangsverstärkers bzw. E3 am Ausgang des Demodulators 5 ein maximaler Leuchtdichte-Bereich von ebenfalls 30 dB an der Bildröhre entspricht.

Das menschliche Auge kann den Leuchtdichtebereich von 30 dB jedoch nur dann auflösen, wenn sein Adaptionszustand nicht durch Umfeldbedingung gestört ist. Ist die Umfeld-Leuchtdichte (Raumhelligkeit) höher als die maximale Leuchtdichte auf dem Bildschirm, so ist der Gesamtkontrast für das Auge zu hoch. In diesem Falle muss die Grundhelligkeit angehoben und der maximale Bildkontrast verringert werden. Speziell zur Kontrasteinstellung ist also ein Kontrastregler 9 mit Kontrasteinstellglied 10 vorgesehen. Das so kontrastgeregelte Echosignal wird dann über einen Video-Endverstärker 11 auf die Bildröhre 12 gegeben. Der Kontrastregler 9 ist so konzipiert, dass er eine lineare Dynamikanpassung des Echosignals an den vom Auge erfassbaren maximalen Bildkontrast gewährleistet. Auf diese Weise lässt sich das Bild an der Bildröhre 12 auf die fürs Auge optimale Grauwertwiedergabe einstellen. Die optimale Einstellung der Grundhelligkeit ist erreicht, wenn die unterste Graustufe gerade noch erkennbar ist. Durch die Kontrastregelung ist also gewährleistet, dass unabhängig von der eingestellten Grundhelligkeit alle Graustufen aufgelöst werden können.

Von besonderer Bedeutung ist die beschriebene Kontrastregelung für die Fotodokumentation mit Hilfe eines Fotovorsatzes 13 (Polaroidkamera). Dieser Fotovorsatz 13 ist im Auslösungsmechanismus über die Signalsteuerleitung 14 mit dem Einstellglied 10 für den Kontrastregler 9 verbunden. Wird der Auslösemechanismus des Fotovorsatzes 13 betätigt, so wird entsprechend über das Einstellglied 10 die Grundhelligkeit am Kontrastregler 9 zu höheren Werten geregelt. Die Abstimmung ist dabei so, dass Grundhelligkeit und Bildkontrast auf den maximalen Kontrast des verwendeten Filmmaterials abgestimmt sind. Mit dieser Art von Kontrastregelung wird eine eindeutig reproduzierbare Foto-Bildqualität gewährleistet, unabhängig davon, wie der Bildbetrachter das von ihm betrachtete Bild am Bildschirm vorhergehend individuell eingestellt hat.

Beim Prinzipschaltbild der Fig. 1 werden die Echosignale E5 am Ausgang des γ-Korrekturgliedes 8 nicht direkt auf den Kontrastregler 9 gegeben. Vielmehr wird dieses Signal E5 zuerst einer Mischstufe 15 zugeleitet, in der dem Signal E5 ein Signal E3' aufgemischt wird, das sich aus der Differentiation des Echosignales E3 (am Ausgang des Demodulators 5) mittels Differenzierstufe 16 ergibt. Das differenzierte Signal E3' wird dem Signal E5 mit etwas erhöhter Leuchtdichte auf dem Bildschirm der Bildröhre 12 überlagert, was zu einem Anheben der Echosignalflanken im Bild führt. Durch geeignetes Mischen des differenzierten und nicht differenzierten Signals in der Mischstufe 15 wird also eine schärfere Darstellung der Organkonturen ermöglicht, ohne dass die Grauwertdarstellung der Strukturechos beeinträchtigt wird.

Ausführungsbeispiele für Schwellenregler 6, $\gamma$-Korrekturglied 8 und Kontrastregler 9 sind in den Fig. 2 bis 4 dargestellt. In der Fig. 2 besteht dabei der Schwellenregler in einfachster Weise aus Komparator 17 mit Schalttransistor 18. Die Bauelemente 19 und 20 sind Beschaltungswiderstände. Mittels eines Potentiometers 21 lässt sich die Schwellspannung S des Schwellenreglers einstellen. Das Potentiometer 21 entspricht also dem Schwellenstellglied 7 der Fig. 1. Die Funktionsweise des Beispiels der Fig. 2 ergibt sich mit dem darüber dargestellten Echosignalverlauf wie folgt:

Das vom Demodulator 5 kommende Echosignal E3 wird im Komparator 17 ständig mit der Schwelle S verglichen. Solange das Signal E3 amplitudenmässig unterhalb der Schwelle S liegt, ist der Transistor 18 leitend, so dass die Störechos kleiner Amplitude vollständig kurzgeschlossen werden. Überschreitet jedoch ein Nutzsignalanteil NE die Schwelle S, so wird der Transistor 18 gesperrt, so dass sich der darunter dargestellte Verlauf E4(t) ergibt. Man sieht sofort, dass die Störechos unterdrückt sind, wobei jedoch die Amplitude der Nutzechos NE unabhängig vom Schwellwert S die gleiche geblieben ist. Damit ist erreicht, dass über der Schwelle S liegende Echos in der Grauwertdarstellung an der Bildröhre unberührt bleiben.

In der Fig. 3 besteht das $\gamma$-Korrekturglied 8 in einfachster Weise aus einer Parallelschaltung von Halbleiterdioden 22, 23 und 24 in Verbindung mit ohmschen Widerständen 25 bis 28 sowie Vorspannungen U01, U02 und U03. Die Vorspannungen U01, U02 und U03 sind gestuft, so dass bei linearem Anstieg des Echosignals E4 über den Bereich O bis 30 dB aufgrund der unterschiedlichen Spannungsschritte, bei denen die Dioden nacheinander leitend werden, sich ein Wurzelfunktionsverlauf über den Bereich 0 bis 15 dB ergibt. Dieser Verlauf ist invers zum $\gamma$-Kennlinienverlauf der Bildröhre.

Der Kontrastregler 9 besteht hingegen gemäss Fig. 4 wiederum aus einem Operationsverstärker 29 mit den Beschaltungswiderständen 30 und 31. Ein Feldeffekttransistor 32, der als variabler Widerstand dient, wird mit dem Steuersignal für die Grundhelligkeit GI beaufschlagt. Bei Normalkontrastregelung lässt sich dabei dieses Signal GI mittels Einstellknopf am Einstellglied 10 direkt einstellen. Bei fotografischer Aufnahme erfolgt hingegen die Aufschaltung des Signals GI über die Signalsteuerleitung 14 bei Auslösen der Aufnahme am Fotogerät 13. Je nach Wahl des Einstellwertes GI für die Grundhelligkeit ist der Transistor 32 mehr oder weniger leitend. Hierdurch erfolgt nicht nur eine Änderung der Grundhelligkeit; vielmehr erfolgt automatisch über den Operationsverstärker 29 auch die lineare Dynamikanpassung des Signals E6 an den vom Auge erfassbaren maximalen Bildkontrast.

**Patentansprüche**

1. Ultraschall-Bildgerät für Ultraschall-Echoaufzeichnung nach dem Impuls-Echo-Prinzip, mit Ultraschall-Sender/Empfänger, der Echosignale mit bestimmtem Dynamikbereich liefert, sowie einer Bildaufzeichnungsvorrichtung für die Echosignale, die eine nichtlineare Leuchtdichte/Ansteuer-Kennlinie aufweist, dadurch gekennzeichnet, dass zwischen Ultraschallempfänger (2) und Bildaufzeichnungsvorrichtung (12) ein Verzerrglied (8) ähnlich oder gleich einem $\gamma$-Korrekturglied, wie es an sich aus der Fernsehtechnik vorbekannt ist, eingeschaltet ist, das die nichtlineare Leuchtdichte/Ansteuer-Kennlinie der Aufzeichnungsvorrichtung (12) in Abhängigkeit von einem vorgebbaren optimalen Dynamikbereich der Echosignale nach vorgebbarem Muster verzerrt, insbesondere linearisiert, und dass dem Verzerrglied (8) ein Schwellenregler (6) vorgeschaltet ist, der in Abhängigkeit von einer vorgebbaren Schwellspannung (S), die oberhalb der Amplitude von Störechos (STE) liegt, solche Signalamplituden (NE), die die Schwellspannung überschreiten, intensitätsgetreu, jedoch unter Unterdrückung der Störechos zum Verzerrglied (8) führt, wozu der Schwellenregler einen Komparator (17) mit zugeordnetem Schalttransistor (18) umfasst, wobei der Transistor (18) bei Signalen (E3) unterhalb der am Komparator eingestellten Schwelle einen Kurzschluss für das Eingangssignal (E3) bildet, der jedoch aufgehoben wird, sobald das einkommende Signal (E3) amplitudenmässig die Schwelle (S) überschreitet.

2. Ultraschall-Bildgerät nach Anspruch 1, dadurch gekennzeichnet, dass dem Schwellenregler (6) ein $\gamma$-Korrekturglied nachgeschaltet ist, das aus einer Parallelschaltung von Schaltdioden (22 bis 24) besteht, die mit unterschiedlichen Spannungen (U01, U02 und U03) in steigenden Stufen vorgespannt sind, so dass ein Linearverlauf des Echodynamikbereiches in einen Funktionsverlauf, vorzugsweise Wurzelfunktionsverlauf, unterschiedlicher Dynamik umgewandelt wird.

3. Ultraschall-Bildgerät nach Anspruch 1 oder 2, dadurch gekennzeichnet, dass in einer Mischstufe (15) das Ausgangssignal (E5) des Verzerrgliedes (8) mit einem Differentiationssignal (E3') überlagert wird, das das mittels Differenzierstufe (16) differenzierte Ausgangssignal (E3) einer Demodulatoreinheit (5) für die Echosignale ist.

4. Ultraschall-Bildgerät nach einem der Ansprüche 1 bis 3, dadurch gekennzeichnet, dass das Ausgangsmaterial des Verzerrgliedes (8) direkt oder nach Durchführung durch einen Mischer (15) über einen Kontrastregler (9) auf eine Bildröhre (12) gegeben ist, der eine Kontrasteinstellvorrichtung (10) aufweist, die wahlweise von Hand oder automatisch in der Grundhelligkeit variierbar ist, wobei bei automatischer Variation diese in Abhängigkeit vom Auslösemechanismus eines Fotoaufnahmegerätes (13) erfolgt in dem Sinne, dass Grundhelligkeit und Bildkontrast auf den maximalen Kontrast des verwendeten Filmmaterials abgestimmt sind.

5. Ultraschall-Bildgerät nach Anspruch 4, dadurch gekennzeichnet, dass sowohl bei Einstellung von Hand als auch bei Einstellung mit Automatik das Einstellglied (10) bei Anheben der Grundhelligkeit den maximalen Bildkontrast entsprechend verringert.

## Claims

1. An ultrasonic video device for ultrasonic echo recording in accordance with the pulse-echo principle, comprising an ultrasonic transmitter/receiver which supplies echo signals of a specified dynamics range and a video recording device for the echo signals which features a non-linear luminous density/drive characteristic, characterised in that a distorter (8) similar to or identical to a γ correcting element as known per se from television technology is connected between the ultrasonic receiver (2) and the video recording device (12), which distorter reshapes and in particular linearises the non-linear liminous density/drive characteristic of the recording device (12) in dependence upon a predeterminable optimum dynamics range of the echo signals in accordance with a predetermined pattern, the distorter (8) being preceded by a threshold regulator (6) utilising a predeterminable threshold voltage (S) which exceeds the amplitude of interference echoes (STE), and supplies the distorter (8) with those signal amplitudes (NE) which exceed the threshold voltage so as to be accurate in terms of intensity yet with suppression of the interference echoes, for which purpose the threshold regulator comprises a comparator (17) with an assigned switching transistor (18), where the transistor (18) forms a short-circuit for the input signal (E3) in the event of signals (E3) below the threshold set on the comparator, which is discontinued as soon as the incoming signal (E3) exceeds the threshold (S) in terms of amplitude.

2. An ultrasonic video device as claimed in Claim 1, characterised in that the threshold regulator (6) is followed by a γ correcting element which consists of a parallel connection of switching diodes (22 to 24) biassed in ascending steps at different voltages (U01, U02, U03) so that a linear curve of the echo dynamics range is converted into a function curve, preferably a root function curve, of different dynamics.

3. An ultrasonic video device as claimed in Claim 1 or 2, characterised in that in a mixing stage (15) the output signal (E5) of the distorter (8) has a differentiation signal (E3') superimposed which represents the output signal (E3) of a demodulator unit (5) for the echo signals, differentiated by means of a differentiator stage (16).

4. An ultrasonic video device as claimed in one of the Claims 1 to 3, characterised in that the output signal of the distorter (8) is fed directly or through a mixer (15) via a contrast regulator (9) to a picture tube (12), which contrast regulator comprises a contrast adjustment device (10) which can be varied in background brightness manually or automatically, automatic variation taking place in dependence upon the trigger mechanism of a photographic camera (13) in that bachground brightness and picture contrast are tuned to the maximum contrast of the film material which is being used.

5. An ultrasonic video device as claimed in Claim 4, characterised in that both in the event of manual adjustment and automatic adjustment, when the background brightness increases the adjustment device (10) reduces the maximum picture contrast accordingly.

## Revendications

1. Appareil à ultrasons pour former des images pour l'enregistrement d'échos par ultrasons suivant le principe d'écho d'impulsion, comportant un émetteur/récepteur d'ultrasons qui délivre des signaux d'écho avec une gamme dynamique déterminée, ainsi qu'un enregistreur d'images pour les signaux d'écho, qui possède une caractéristique luminance/commande non linéaire, caractérisé par le fait qu'entre le récepteur d'ultrasons (2) et l'enregistreur d'images (12) est inséré un élément de distorsion (8) similaire ou identique à un élément de correction γ, tel que connu en soi d'après la technique de télévision, qui déforme suivant un modèle pouvant être prédéterminé, notamment linéaire, la caractéristique luminance/commande non linéaire de l'enregistreur d'images (12) en fonction d'une gamme dynamique optimale pouvant être prédéterminée, des signaux d'écho, et qu'en amont de l'élément de distorsion (8) est branché un régulateur de seuil (6) qui, en fonction d'une tension de seuil (5) pouvant être prédéterminée, qui est supérieure à l'amplitude d'échos parasites (STE), applique à l'élément de distorsion (8) les amplitudes de signaux (NE) qui sont supérieures à la tension de seuil, sans en modifier l'intensité mais en supprimant les échos parasites, ce pour quoi le régulateur de seuil comporte un comparateur (17) auquel est associée un transistor de commutation (18), le transistor (18) formant un court-circuit pour le signal d'entrée (E3) dans le cas de signaux (E3) inférieur au seuil réglé au niveau du comparateur, court-circuit qui est cependant supprimé dès que le signal (E3) qui arrive dépasse le seuil (S) en amplitude.

2. Appareil à ultrasons pour former des images suivant la revendication 1, caractérisé par le fait qu'en aval du régulateur de seuil (6) est branché un élément de correction γ, qui est contitué par un montage en parallèle de diodes de commutation (22 à 24), qui sont polarisées de façon croissante par des tensions différentes (U01, U02, U03), de manière qu'une allure linéaire de la gamme dynamique d'écho soit transformée en allure de fonction, de préférence une allure de fonction de racine, de dynamique différente.

3. Appareil à ultrasons pour former des images, suivant l'une des revendications 1 ou 2 caractérisé par le fait que, dans un étage mélangeur (15), au signal de sortie (E5) de l'élément de distorsion (8) est superposé un signal de différentiation (E3') qui est le signal de sortie (E3), différentié au moyen d'un étage différentiateur (16), d'une unité de démodulation (5) pour les signaux d'écho.

4. Appareil à ultrasons pour former des images, suivant l'une des revendications 1 à 3, caractérisé par le fait que le signal de sortie de l'élément de distorsion (8) est appliqué, directement ou après passage dans un mélangeur (15), à un tube image, par l'intermédiaire d'un régulateur de contraste (9), tube image qui comporte un dispositif de réglage de contraste (10) dont la luminosité moyenne peut être modifiée au choix manuellement ou automatiquement, dans le cas d'une variation automatiquement, dans le cas d'une variation automatique celle-ci étant effectuée, en fonction du mécanisme de déclenchement d'un appareil de prise de vues photographiques (13) de manière à adapter la luminosité moyenne et le contraste d'image au contraste maximal du matériau du film utilisé.

5. Appareil à ultrasons pour former des images, suivant la revendication 4, caractérisé par le fait qu'aussi bien dans le cas d'un réglage manuel que dans le cas d'un réglage automatique, l'élément de réglage (10) réduit le contraste d'image maximal de façon correspondante lors d'une augmentation de la luminosité moyenne.

**FIG 1**

**FIG 2**

**FIG 3**

**FIG 4**